**Europäisches Patentamt**

**European Patent Office**

(19)

**Office européen des brevets**

(11) Veröffentlichungsnummer : **0 068 191 B2**

(12) **NEUE EUROPÄISCHE PATENTSCHRIFT**

(45) Veröffentlichungstag der neuen Patentschrift :
11.09.91 Patentblatt 91/37

(51) Int. Cl.$^5$ : **A61K 9/20, A61K 31/505**

(21) Anmeldenummer : **82105000.2**

(22) Anmeldetag : **08.06.82**

(54) **Orale Dipyridamolformen.**

Die Akte enthält technische Angaben, die nach
dem Eingang der Anmeldung eingereicht
wurden und die nicht in dieser Patentschrift
enthalten sind.

(30) Priorität : **19.06.81 DE 3124090**

(43) Veröffentlichungstag der Anmeldung :
**05.01.83 Patentblatt 83/01**

(45) Bekanntmachung des Hinweises auf die
Patenterteilung :
**08.01.86 Patentblatt 86/02**

(45) Bekanntmachung des Hinweises auf die
Entscheidung über den Einspruch :
**11.09.91 Patentblatt 91/37**

(84) Benannte Vertragsstaaten :
**AT BE CH DE FR IT LI LU NL SE**

(56) Entgegenhaltungen :
**EP-A- 0 032 562**
**DE-A- 2 146 859**
**DE-A- 2 157 201**
**DE-A- 2 700 433**
**FR-A- 2 390 959**
**FR-A- 2 430 766**

(56) Entgegenhaltungen :
**GB-A- 2 039 737**
**Cortot "Gastric Emptying of Solid Dosage
Forms", Pharmacy International, Sept. 1984,
S. 228-231**
**S.S. Davis et al., International Journal of Pharmaceutics, 21(1984), S. 331-340**
**J.T. Fell, G.A. Digenis, International Journal of
Pharmaceutics, 22 (1984), S. 1-15**
**Japanisches Prospekt für Anginal mit englischer Uebersetzung**
**S.S. Davis, J.G. Hardy, J.W. Fara, Gut 27,
(1986), S. 886**

(73) Patentinhaber : **Dr. Karl Thomae GmbH
Postfach 1755
W-7950 Biberach (Riss) (DE)**

(72) Erfinder : **Brickl, Rolf, Dr. Dipl.-Chem.
Erlenweg 37
W-7951 Warthausen (DE)**
Erfinder : **Gruber, Peter, Dr. Dipl.-Chem.
Wetterkreuzstrasse 36
W-7950 Biberach 1 (DE)**
Erfinder : **Schepky, Gottfried, Dr.
Ulrich-von-Hutten-Weg 2
W-7950 Biberach 1 (DE)**
Erfinder : **Bozler, Gerhard, Dr. Dipl.-Chem.
Alpenstrasse 38
W-7950 Biberach 1 (DE)**

EP 0 068 191 B2

EP 0 068 191 B2

## Beschreibung

Die Erfindung betrifft neue orale Dipyridamolformen mit einer unverzögerten Freigabe des Wirkstoffes und einer relativen Bioverfügbarkeit von über 100 % bezogen auf Dipyridamollösungen und wesentlich geringeren inter- und intraindividuellen Blutspiegelschwankungen bzw. die Verwendung solcher Darreichungsformen in Form von Pellets und Granulaten zur Herstellung von fertigen Arzneiformen mit rascher Wirkstoff-Freigabe, wobei diese fertigen Arzneiformen sowohl geschmacksabdeckende Kapseln, die mit diesen Pellets oder Granulaten gefüllt sind, als auch Pellets, Granulate oder Tabletten, die jeweils mit einem geschmacksabdeckenden Überzug versehen sind, umfassen. Diese neuen galenischen Zubereitungsformen bieten in pharmakokinetischer Hinsicht gegenüber den bisher bekannten galenischen Formen besondere Vorteile.

Das Dipyridamol (2,6-Bis(dieäthanolamino)-4,8-dipiperidiniopyrimido[5,4-d]pyrimidin) stellt einen seit Jahren bewährten Wirkstoff dar. Die bisher bekannten, diesen Wirkstoff enthaltenden galenischen Zubereitungen weisen wegen der speziellen physikalischen Eigenschaften des Dipyridamols für bestimmte Anwendungszwecke einige Nachteile auf.

Durch die FR-A-2 430 766 wird eine Dipyridamol und saure Hilfsstoffe enthaltende Retardform beschrieben, deren Wirkstoff-Freigabe durch eine besondere Diffusionshülle gesteuert wird. Die Freigabe des Wirkstoffes erfolgt während einer Dauer von beispielsweise 8 Stunden; eine derartige Retardform eignet sich nicht für solche Fälle, bei welchen ein so-fortiger, sehr hoher Blutspiegel an Dipyridamol anzustreben ist. In der FR-A-2 390 959 werden synergistisch wirkende Retardformen aus Dipyridamol und Acetylsalizylsäure beschrieben. Zwischen der Acetylsalizylsäure und dem Dipyridamol soll eine gegenseitige synergistische Beeinflussung stattfinden; diese dieses Gemisch enthaltende Retardformen sind aber so aufgebaut, daß die beiden Wirkstoffe Acetylsalicylsäure und Dipyridamol sich nicht direkt berühren. Ein derartiger Aufbau der Darreichungsform, der nach außen hin mit einer retardierend wirkenden Dialysenmembran versehen ist, ist für die Erzielung einer rasch einsetzenden Wirkung des Dipyridamols überhaupt nicht geeignet, auch dann nicht, wenn die Acetylsalicylsäure in Wegfall käme.

Da Dipyridamol nur im sauren Milieu gut wasserlöslich ist, kann es aus festen galenischen Formen nur dann in Lösung gehen und resorbiert werden, wenn die galenischen Zubereitungen sich ausreichend lange im sauren Bereich befinden, d. h. die Löslichkeit und damit auch die Resorption hängen stark von der Verweilzeit und dem pH-Wert im Magen und oberen Intestinaltrakt ab. Dies führt zu starken inter- und intraindividuellen Schwankungen der Blutspiegel (siehe Tabelle 1), da Motilität des Probanden, pH-Wert des Patienten in Magen und Darm und Nahrungsaufnahme einen starken Einfluß auf die Resorption ausüben. Bei einigen wenigen Patienten sind die Blutspiegel sogar so niedrig, daß man praktisch von einer fehlenden Resorption sprechen kann.

Es wurde nun eine neue, oral zu verabreichende und den Wirkstoff rasch freisetzende Dipyridamol-Zubereitung gefunden, die dadurch charakterisiert ist, daß sie auf 1 Mol Dipyridamol oder auf 1 Mol von dessen Säureadditionssalz in enger Vermischung mindestens 5 Val eines oral verträglichen, sauren Hilfsstoffes, gegebenenfalls neben üblichen sonstigen Zusatzstoffen, enthält. Diese Dipyridamolzubereitung zeichnet sich dadurch aus, daß sie unabhängig von den physiologischen Verhältnissen des Magen-Darmtraktes (beispielsweise unabhängig vom pH-Wert, der Pufferkapazität und der Motilität dieses Traktes) zu einer reproduzierbaren hohen Bioverfügbarkeit des Dipyridamols führt. Die Verwendung der unverzögert den Wirkstoff freigebenden, erfindungsgemäßen Darreichungsformen zur Erzielung gleichbleibender Blutspiegel bei Patienten, gleichgültig ob diese Patienten gute oder schlechte Absorber für den Wirkstoff Dipyridamol sind, ist neu und nicht vorhersehbar.

An sich müsste in dieser Hinsicht eine saure Dipyridamollösung die beste Darreichungsform sein, da hierbei der Wirkstoff bereits in gelöster Form angeboten wird; es wäre damit eine vollständige Resorption und Hand in Hand eine sehr hohe Bioverfügbarkeit zu erwarten. Aus diesem Grund ist es üblich, die Qualität einer galenischen Formulierung in Form ihrer relativen Bioverfügbarkeit im Vergleich zur Lösung der Substanz anzugeben. Dazu wird die Fläche unter der Blutspiegelkurve (= AUC) bestimmt und mit derjenigen der Lösung (= 100%) verglichen. Es wurde aber in völlig überraschender Weise gefunden, dass die relative Bioverfügbarkeit von Dipyridamol bei Anwendung der erfindungsgemässen festen Formen im Vergleich zu der Anwendung einer sauren Dipyridamollösung oberhalb der theoretisch möglich anzusehenden Grenze von 100%, nämlich bei 140 bis 150%, liegt.

Versuche mit radioaktiv markiertem Dipyridamol, bei denen die Substanz einmal intravenös als saure Lösung gegeben wurde und das andere Mal peroral als saure Lösung, lassen folgenden Schluss zu:

Dipyridamol wird, auch wenn es dem Körper in gelöster Form zur Verfügung gestellt wird, nicht vollständig, sondern nur zu etwa 60-70% resorbiert. Auf diese Resorptionsquote kann einerseits aus dem Vergleich der Urinausscheidung bei beiden Applikationen, andererseits aber auch aus Clearance-Berechnungen geschlossen werden.

2

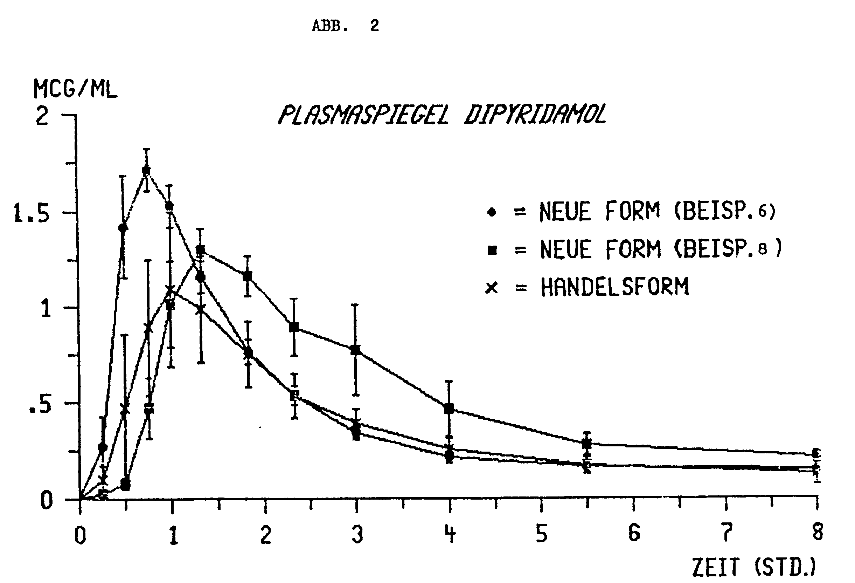

In der GB-A 2 039 737 A wird im Beispiel 8 ein Granulat, bestehend aus 0,5 kg Dipyridamol und 0,25 kg Fumarsäure, und dessen Herstellung beschrieben. Es kommen hierbei auf 1 Mol Dipyridamol 4 Val Fumarsäure. Eine Nacharbeitung dieses Beispiels ergab ein Dipyridamol-Granulat, dessen pH-unabhängige Löslichkeit vollkommen unbefriedigend war, obwohl die Fumarsäure in einem Überschuss von 1 Mol Dipyridamol zu 4 Val Fumarsäure vorlag. Wie die Abb. 2 der genannten Anmeldung ausserdem zeigt, beträgt die Wirkstofffreigabe nach einer Stunde nur 10% und nach 6 Stunden erst ca. 100%. Daraus und aus der dort beschriebenen Art und Weise der Herstellung ergibt sich, dass es sich um eine Retardform für Dipyridamol handelt. Die erfindungsgemässen Zubereitungsformen unterscheiden sich von diesen dadurch, dass sie nach ca. 30 Minuten bis 2 h nach Einnahme zu gut reproduzierbaren Blutspiegeln bei höchstmöglicher Bioverfügbarkeit führen.

Die erfindungsgemässen Formen zeichnen sich demgegenüber dadurch aus, dass in ihnen eine innige Mischung von Dipyridamol oder von dessen Säureadditionssalz und von einem Überschuss im Verhältnis von 1 Mol Dipyridamol zu mindestens 5 Val eines physiologisch unbedenklichen sauer reagierenden Stoffes vorliegt. Es war nicht zu erwarten, dass ein weiterer Überschuss an Säure plötzlich zu einer signifikanten Steigerung der Bioverfügbarkeit des Dipyridamols führt. Als Obergrenze gelten 30 Val des sauren Hilfsstoffes, da darüber gehende Mengen an saurem Hilfsstoff zu Formen führt, die nicht mehr schluckbar sind.

Die Abhängigkeit von Löslichkeit bzw. Auflösegeschwindigkeit des Dipyridamols von der Menge an zugesetzter Säure pro Dosis wurden daraufhin untersucht; die beiden nachstehenden Tabellen zeigen diese Abhängigkeit am Beispiel von Dipyridamol-Filmtabletten mit unterschiedlichen Zusätzen an Weinsäure bzw. Fumarsäure pro Tablette. Die Tabletten wurden in vitro nach der USP XX-paddle-Methode bei 100 U/min. in 500 ml verdünntem Mc Ilvain-Puffer von pH 6 untersucht.

In-vitro-Freigabegeschwindigkeit aus Dipyridamol-Filmtabletten mit unterschiedlichem Fumarsäurezusatz in verdünnter Pufferlösung von pH 6 (75 mg Dipyridamol/Tablette)

| | % freigesetztes Dipyridamol nach Zusatz von | | | | |
|---|---|---|---|---|---|
| nach Minuten | 0 mg | 30 mg | 60 mg | 120 mg | 180 mg Fumarsäure/Tablette |
| 5 | 7 | 17 | 37 | 53 | 66 |
| 10 | 7 | 27 | 60 | 77 | 85 |

In-vitro-Freigabegeschwindigkeit aus Dipyridamol-Filmtabletten mit unterschiedlichem Weinsäurezusatz in verdünnter Pufferlösung von pH 6 (75 mg Dipyridamol/Tablette)

| | % freigesetztes Dipyridamol nach Zusatz von | | | | |
|---|---|---|---|---|---|
| nach Minuten | 0 mg | 10 mg | 20 mg | 30 mg | 40 mg | 80 mg Weinsäure/Tablette |
| 10 | 7 | 13 | 21 | 35 | 33 | 39 |
| 30 | 7 | 13 | 21 | 40 | 50 | 68 |

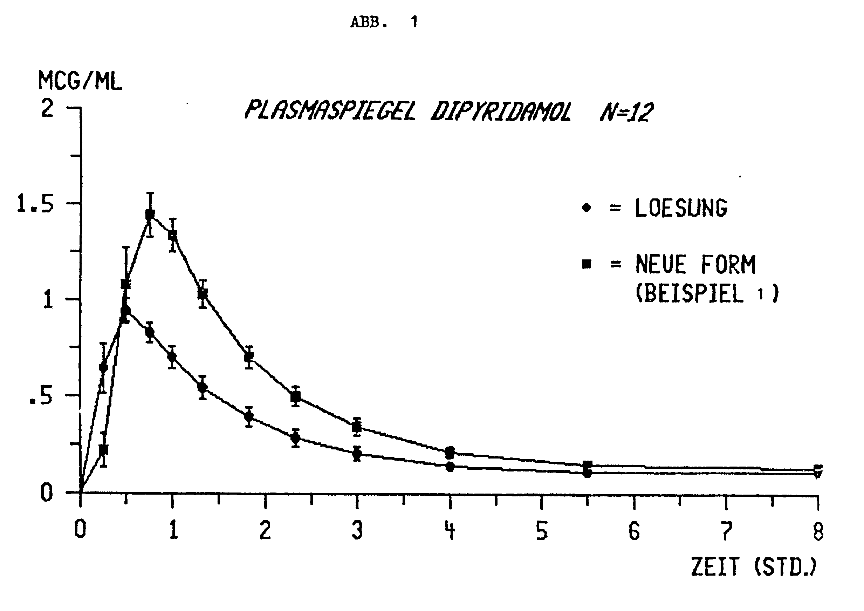

Es war nicht vorhersehbar, dass Dipyridamol, wenn es in nur relativ kleinen absoluten Mengen vorliegt und vollständig gelöst ist, stark übersättigte Lösungen, die bis zur 20fachen Konzentration der wahren Löslichkeit reichen, bildet und dass dieses Verhalten besonders dann eintritt, wenn mehr als 5 Val einer sauer reagierenden Substanz pro Mol Dipyridamol vorliegt. In-vivo Versuche mit den neuen erfindungsgemässen Formen (siehe Abb. 1 und 2) zeigen, dass im Vergleich zu sauren Lösungen bei der Applikation der erfindungsgemässen Formen eine relative Bioverfügbarkeit von etwa 150% erreicht wird. Ein weiterer Vorteil dieser Formen besteht darin, dass infolge der pH-unabhängigen Löslichkeit das Freigabeprofil und damit auch die Blutspiegel gesteuert werden können, ohne dass ein Verlust an Bioverfügbarkeit eintritt.

Der ausserordentlich überraschende Befund der Steigerung der relativen Bioverfügbarkeit gegenüber der Lösung auf ca. 150% kann möglicherweise daran liegen, dass sich übersättigte, höher konzentrierte Wirkstoflösungen bilden. Bei der Einnahme von Dipyridamol in gelöster Form ist es nicht ausgeschlossen, dass Teile des Wirkstoffes im oberen Darmabschnitt aus der Lösung bereits ausgefallen sind, bevor sie vollständig resorbiert wurden, was überraschenderweise beim Vorliegen übersättigter Lösungen nicht der Fall sein dürfte. Man muss sich stets vor Augen halten, dass beim Übergang vom sauren Magen zum Darm die Löslichkeit des Dipyridamols weit mehr als um den Faktor 1000 abnimmt und z.B. bei pH 7.0 nur noch ca. 1 mg pro Liter beträgt. Ab ca. pH 4.0 ist die Löslichkeit des Dipyridamols bereits so gering, dass praktisch kein Blutspiegel mehr aufgebaut wird.

Da Dipyridamol eine reversibel wirkende Substanz ist, d.h. therapeutische Wirksamkeit nur besteht, solange ausreichend hohe Blutspiegel aufrecht erhalten werden, ist damit eine weitere Verbesserung der therapeutischen Wirkung gegeben. Insgesamt weisen die erfindungsgemässen neuen galenischen Formen damit gegenüber konventionellen Formulierungen folgende Vorteile auf:

1. Höhere Bioverfügbarkeit

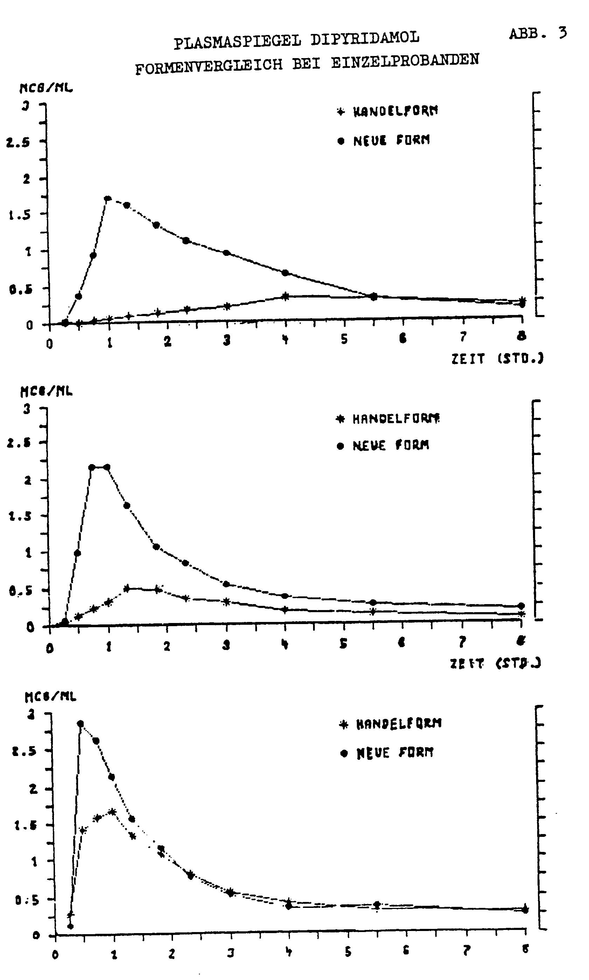

2. Höhere therapeutische Sicherheit, die dadurch erreicht wird, dass einerseits die inter- und intraindividuellen Schwankungen, die für bekannte Formen typisch sind, verkleinert werden und zum anderen völlig unzureichende Blutspiegelwerte nicht auftreten (beispielsweise bei Patienten, die normalerweise Dipyridamol kaum resorbieren, siehe Abb. 3).

3. Durch Steuerung der Freigabe kann eine Verbesserung der therapeutischen Wirkung und Vermeidung von Nebenwirkungen bei höheren Dosierungen erreicht werden (siehe Abb. 2).

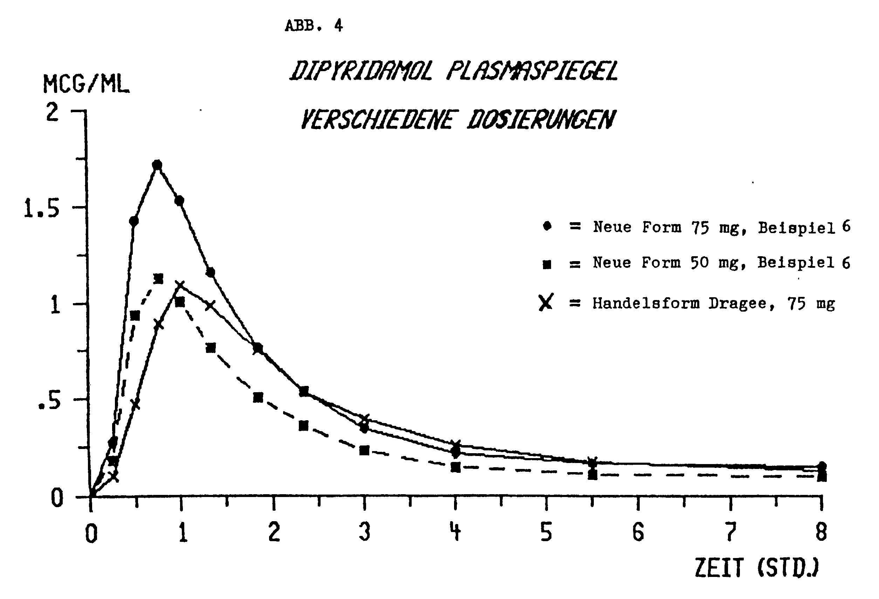

4. Die höhere Bioverfügbarkeit ermöglicht in einigen Fällen eine Reduzierung der Dosis (Abb. 4 zeigt, dass eine Reduktion der Dosis auf 50 mg noch eine bioäquivalente Form ergibt).

Es hat sich gezeigt, dass die bemerkenswerte Steigerung der relativen Bioverfügbarkeit des Dipyridamols mit einer Vielzahl von Arzneiformen für die orale Anwendung realisierbar ist. Wesentlich für optimale Blutspiegel ist die Wahl und das richtige Verhältnis von saurem Hilfsstoff zu Dipyridamol.

Eingehende Untersuchungen haben gezeigt, dass mindestens 5 Val saurer Hilfsstoff für 1 Mol Dipyridamol benötigt werden, um eine deutliche Verbesserung der Bioverfügbarkeit des Dipyridamols zu erreichen. Die Menge an saurem Hilfsstoff im Verhältnis zum Dipyridamol ist an sich nach oben hin nicht begrenzt; sie wird nur dadurch limitiert, dass bei zu grosser Menge keine mehr gut schluckbare orale Form des Dipyridamols realisierbar ist. Bevorzugt ist ein Verhältnis von 10 bis 30 Val saurer Hilfsstoff zu 1 Mol Dipyridamol. Als Säureadditionssalze des Dipyridamols kommen physiologisch verträgliche Additionsprodukte mit anorganischen oder organischen Säuren in Betracht. Geeignete Säuren sind beispielsweise Salzsäure, Weinsäure oder Zitronensäure. Als saure Hilfsstoffe eignen sich eine ganze Reihe von pharmazeutisch unbedenklichen organischen Genusssäuren und organischen und anorganischen Salzen wie z.B. Zitronensäure, Weinsäure, Äpfelsäure, Ascorbinsäure, Fumarsäure, Bernsteinsäure, Adipinsäure, Monokalium- und Natriumsalze der genannten Säure wie z.B. Monokaliumcitrat und Monokaliumtartrat aber auch Natriumhydrogensulfat oder Betainhydrochlorid. Gelegentlich können auch Anhydride wie das Bernsteinsäureanhydrid, Glutarsäureanhydrid oder D-Glucuronsäure-$\gamma$-lacton, die in Gegenwart von Wasser zu Säuren hydrolysieren, zur Erhöhung der Löslichkeit des Dipyridamols herangezogen werden. Bevorzugt werden Zubereitungsformen, die Fumarsäure enthalten. Diese Zubereitungsformen zeichnen sich durch eine hohe Lagerstabilität aus.

Die Einfachheit, mit der optimale orale feste Dipyridamol-Arzneiformen realisiert werden können, war nicht vorhersehbar und für den Fachmann überaus überraschend. Im Sinne der Erfindung werden z.B. bevorzugte Dipyridamol-Formen dadurch hergestellt, dass in einfacher Weise Dipyridamol oder dessen Säureadditionssalz und Fumarsäure mit und ohne Bindemitteln vermischt und dann über Tablettenpressen oder Walzenkompaktoren verdichtet werden. Die verdichteten Körper werden anschliessend über Trockengranuliergeräte zur Abfüllung in Hartgelatinekapseln wieder aufgebrochen. Zur Erreichung optimaler Blutspiegel spielen die Menge der sauren Hilfsstoffe, die Art der sauren Hilfsstoffe und das Korngrössenspektrum des Trokkengranulates die entscheidende Rolle.

Andererseits kann aber auch Dipyridamol oder dessen Säureadditionssalz zusammen mit den sauren Hilfsstoffen pelletiert werden, wobei die Pellets mit einem Durchmesser von 0,1 bis 2,0 mm (vorzugsweise 0,5 bis 1,0 mm) ausgesondert und in Hartgelatinekapseln abgefüllt werden.

Die Dipyridamol-Granulate und die Pellets lassen sich anschliessend mit einem Lack überziehen, der im Gastrointestinaltrakt mindestens 90% des Wirkstoffes über einen Zeitraum von 2 Stunden verteilt freigibt.

Der Wirkstoff lässt sich auch mit dem sauren Hilfsstoff sowie weiteren direkt tablettierbaren Hilfsstoffen sowie dem Schmiermittel zu einer direkt tablettierbaren Mischung verarbeiten, die dann zu Tablettenkernen verpresst wird, wobei anschliessend diese mit einem Lack oder geschmacksabdeckenden Überzug versehen werden.

Natürlich kann man auch zuerst den Wirkstoff mit einem bzw. mehreren sauren Hilfsstoffen zuerst feucht oder trocken granulieren, wobei nach Zumischung weiterer Hilfsstoffe die Granulate zu Tablettenkernen verpresst werden. Man kann aber auch den Wirkstoff mit üblichen Hilfsstoffen zuerst durch Feucht- oder Trockengranulation in ein Granulat überführen, dem nachträglich die sauren Hilfsstoffe sowie ein Schmiermittel zugesetzt werden; erst dann wird die Masse zu Tablettenkernen verpresst.

Als besonders geeignete Säure erweist sich die Fumarsäure. Sie ist eine physiologisch völlig unbedenkliche Säure, gut verpressbar und erzeugt in Verbindung mit Dipyridamol keine hygroskopischen Gemische. Erfindungswesentlich ist ihre geringe Löslichkeit; sie sorgt dafür, dass im Gastrointestinaltrakt um das Granulatkorn stets eine ausreichende saure Mikrosphäre vorhanden ist, in der sich das schwerlösliche Dipyridamol sicher und vollständig auflöst.

Wird aus medizinischen Gründen ein in seiner Höhe reduziertes, dafür aber verbreitertes Blutspiegelmaximum gewünscht, so gibt es eine ganze Reihe von galenischen Möglichkeiten. Wie die Beispiele 1 und 2 zeigen, führt eine Erhöhung des Säurezusatzes zu einer Beschleunigung der Dipyridamol-Freigabe, eine Verringerung dagegen (Beispiel 2) zu einer Verlangsamung der Wirkstofffreigabe.

Weitere Ausführungsformen für Dipyridamol-Granulate unterscheiden sich in der Weise, dass neben gut wasserlöslichen Bindemitteln wie PVP auch in Gegenwart von Wasser schleimbildende Hilfsstoffe oder sogar wasserabstossende Hilfsstoffe dem Dipyridamol und dem sauren Hilfsstoff zugemischt werden können. Wie die Wirkstofffreigabeergebnisse der Beispiele 3 und 4 beweisen, tritt zum Teil eine merkliche Verzögerung der Wirkstofffreigabe bis auf ca. 2 h ein.

Ist dagegen ein sehr rasch ansteigender Blutspiegel für das Dipyridamol gewünscht, so kann man neben einer Erhöhung des Säurezusatzes oder einer Verminderung der Granulatteilchengrösse (Vergrösserung der Oberfläche) besonders vorteilhaft die Art der Säure ändern. Aufgrund der hohen Löslichkeiten insbesondere von Weinsäure, Zitronensäure, Äpfelsäure, Ascorbinsäure usw. löst sich in vitro das Dipyridamol in weniger als 5 Minuten vollständig und unabhängig vom pH-Wert des Freigabemilieus auf (vgl. Beispiel 6).

Dipyridamol- oder Dipyridamol-Säureadditionssalz-Säurekombinationen lassen sich auch als Tabletten auf überraschend einfache Weise realisieren. Es zeigte sich, dass auch in Gegenwart üblicher Tablettenhilfsstoffe der Komprimiervorgang während der Tablettierung ausreicht, um eine genügend innige mechanische Verbindung zwischen Wirkstoff und zugesetzter Säure zu bewirken. Die Tablettenbeispiele (vgl. Tabellen auf Seite 5) mit 40 bzw. 80 mg Weinsäure auf jeweils 75 mg Dipyridamol (Beispiel 10) verdeutlichen dies, ebenso wie die Beispiele 11 und 12, die bei gleichem Wirkstoffgehalt 60 bzw. 120 mg Fumarsäure enthalten. Aus Geschmacksgründen wurden die Tabletten jeweils mit einem dünnen Hydroxypropyl-methylcellulose-Überzug versehen. Zur besseren Verarbeitbarkeit (gegen hygroskopisches Verhalten) wurde im Beispiel 10 überzogene Weinsäure eingesetzt. In-vitro-Dissolutionstests hatten keine signifikanten Unterschiede zwischen dieser Ware und nicht überzogener Weinsäure ergeben.

Ohne im einzelnen weitere Beispiele aufzuführen, ist es dem Fachmann leicht verständlich, dass durch die Art und Menge der zugesetzten Hilfsstoffe, die Art und Menge an saurem Hilfsstoff, die Art der Herstellung (Granulat-Teilchengrösse) die Dipyridamol-Wirkstofffreigabe in weiten Grenzen den medizinischen Anforderungen angepasst werden kann.

So eignen sich neben den verwendeten Hilfsstoffen Polyvinylpyrrolidon, hydriertes Rizinusöl und Polyacrylsäure genauso auch Hilfsstoffe wie Methyl-, Äthyl-, Hydroxyäthyl- oder Hydroxypropylmethyl-cellulose. Ausserdem können zur Erreichung der gewünschten Freigabe die Mischungen, bestehend aus Dipyridamol oder aus dessen Säureadditionssalz und sauren Stoffen, mit in organischen Lösungsmitteln gelösten Fetten oder magensaftresistenten Lacken wie Celluloseacetatphthalat, Schellack, Hydroxypropylmethylcellulosephthalat granuliert und dann verpresst und wieder in Granulate gebrochen werden.

Sind aus therapeutischen Gründen höhere Dipyridamol-Dosierungen notwendig und hohe Blutspiegelspitzen wegen eventueller Nebenwirkungen zu vermeiden, so gelingt dies erfindungsgemäss besonders durch die Formen, wie sie in den Beispielen 8 und 9 beschrieben sind. Bei Dosierungen, z.B. über 100 mg Dipyridamol, gelingt mit diesen Formen statt der Erreichung sehr hoher Blutspiegelspitzen die Verbreiterung des Blutspiegelmaximums. Da diese Formen den Wirkstoff kontrolliert, z.B. zwischen 1 und 2,0 Stunden, abgeben, befinden sich die kleinen Teilchen daher bereits zum grössten Teil im Duodenum, d.h. in einem pH-Bereich über pH 4.0.

Deshalb müssen diese Formen den Wirkstoff in einem pH-Milieu freisetzen, in dem der Wirkstoff im biologischen Sinne praktisch nicht mehr löslich ist. Verzögert man die Dipyridamol-Freigabe weiter, d.h. kommen die kleinen Dipyridamol-Einheiten in tiefere Darmabschnitte, so ist eine vollständige Auflösung und Resorption nicht mehr gewährleistet. Das Optimum an Resorption des Dipyridamols wird dadurch erreicht, dass im Sinne der Erfindung die Zusammenhänge zwischen der Art und Menge an saurem Hilfsstoff, der Art der Zuschlagstoffe, der Art der Verarbeitung, der eingestellten Wirkstofffreigabe genau bestimmt wurden.

In-vivo-Prüfung der erfindungsgemässen Formen an Menschen:

Alle Prüfungen wurden an gesunden, freiwilligen Probanden, meist in Form von Cross-over-Versuchen ausgeführt. Da Dipyridamol nur zu einem sehr geringen Teil im Urin ausgeschieden wird, wurde als biologisch relevanter Parameter ausschliesslich der Plasmaspiegel, der durch Fluoreszenzmessung bestimmt wurde, herangezogen. Es wurden die galenischen Formen der Beispiele 1, 6, 8, 10, 11 und 12 am Menschen überprüft.

Da jedoch in vivo die erfindungsgemässen neuen, oralen und unverzögerten Formen sich gegenseitig nicht signifikant unterscheiden, werden in den folgenden Abbildungen nur die Ergebnisse mit den Beispielen 1, 6 und 8 gezeigt.

Abb. 1 zeigt die Plasmaspiegel von 12 Probanden nach Gabe von 75 mg Dipyridamol-Lösung im Vergleich zu einer Kapsel nach Beispiel 1. Es zeigt sich, dass nach einer kurzen Verzögerung (lag time), die vermutlich durch das Auflösen der Kapsel bedingt ist, bei den neuen Formen im Mittel erheblich höhere Werte auftreten.

Abb. 2 zeigt, dass bei einer gesteuerten Freigabe die Blutspiegelmaxima nur unwesentlich höher sind, als dies bei den derzeitigen Handelsformen der Fall ist. Dieses Maximum ist jedoch wesentlich länger anhaltend, d.h. Dipyridamol kann in derartigen Formen erheblich länger therapeutisch wirken, ohne dass das Risiko von Nebenwirkungen erhöht wird.

Bei Betrachtung der Plasmaspiegel der Einzelprobanden zeigt sich, dass vor allem bei niedrigen Werten der Handelsformen und den gelegentlich auftretenden « Non-absorbern » eine sehr starke Erhöhung des Plasmaspiegels auftritt (siehe Abb.3), während bei Probanden die bereits mit den Handelsformen gute Plasmaspiegel aufweisen, nur eine relativ geringe Erhöhung eintritt. Dadurch werden die sonst üblichen Plasmaspiegelschwankungen, die eine Therapieerschwernis darstellen, deutlich verringert. Dies zeigt auch ein Vergleich der Variationskoeffizienten von Handelsform und erfindungsgemässer Form in Tabelle 1: Mit Ausnahme der Werte bei 0,25 Stunden (dieser Wert wird durch die Auflösung der Kapsel bestimmt) weisen die Variationskoeffizienten der neuen Formen um ca. 60% niedrigere Werte auf.

Tabelle 1:
Vergleich der Variationskoeffizienten von Handelsform auf neue Form:

| Zeitpunkt | Variationskoeffizienten % (Standard durch Mittelwert) | |
|---|---|---|
| | Handelsform | neue Form |
| 0,25 | 92,4 | 141,1 |
| 0,50 | 120,9 | 45,0 |
| 0,75 | 63,2 | 15,8 |
| 1,00 | 59,6 | 17,0 |
| 1,33 | 58,1 | 18,5 |
| 1,83 | 54,7 | 20,7 |
| 2,33 | 55,4 | 23,3 |
| 3,00 | 50,6 | 31,0 |
| 4,00 | 60,6 | 31,0 |
| 5,50 | 61,4 | 43,6 |
| 8,00 | 61,6 | 28,1 |

Abb. 4 zeigt, dass bei den neuen Formen bei einer Dosisreduktion von 75 mg auf 50 mg, also um 33%, Plasmaspiegel, die denen der derzeitigen Handelsform entsprechen, auftreten.

In den Zeichnungen bedeutet:
MCG/ML  =Mikrogramm pro Milliliter
MG        =Milligramm

Die folgenden Beispiele sollen die Erfindung näher erläutern:

Beispiel 1

Man mischt 3 kg Dipyridamol mit 6,3 kg Fumarsäure und befeuchtet die Mischung mit 2 kg einer 15%igen alkoholischen Polyvinylpyrrolidonlösung. Nach dem Trocknen bei 40°C und Sieben mischt man das Granulat mit 30 g Polyvinylpyrrolidonpulver und 100 g Magnesiumstearat. Die zu grossen Tabletten verpresste Mischung wird in einem Trockengranuliergerät gebrochen und gesiebt. Verwendung findet die Granulatfraktion von 0,4 bis 1,0 mm, die in Hartgelatine-Steckkapseln abgefüllt wird.

In-vitro-Wirkstofffreigabe Dipyridamol:

Bedingungen: USP XX-paddle-Methode, 100 Umdrehungen/Minute 37°C (Wenn nicht anders erwähnt, erfolgte die in-vitro-Freigabe stets unter diesen Bedingungen)

pH 1.2    USP-Magensaft
pH 4.0 |  Mc-Ilvain-Puffer
pH 6.0 |

Einwaage 75 mg (bezogen auf Dipyridamol)

Wirkstofffreigabe Dipyridamol:

| pH-Wert | 50% | >90% |
|---------|---------|---------|
| 1,2 | 3 Min. | 8 Min. |
| 4,0 | 4 Min. | 12 Min. |
| 6,0 | 5 Min. | 15 Min. |

Beispiel 2

45 kg Dipyridamol, 45 kg Fumarsäure und 9 kg Polyvinylpyrrolidon mischt man 20 Minuten in einem Kubusmischer. Man fügt 0,5 kg Magnesiumstearat zu und mischt nochmals für 5 Minuten. Die Mischung gibt man über einen Walzenkompaktor, dem ein Trockengranuliergerät mit Siebeinrichtung nachgeschaltet ist. Verwendung findet die Fraktion 0,4 bis 1,0 mm. Feinere Anteile werden zurückgeführt und erneut verdichtet.

Das Granulat füllt man je nach gewünschter Dosierung in entsprechend geeignete Hartgelatine-Steckkapseln ab.

Wirkstofffreigabe Dipyridamol:

| pH-Wert | 50% | >90% |
|---------|---------|---------|
| 1,2 | 6 Min. | 12 Min. |
| 4,0 | 7 Min. | 20 Min. |
| 6,0 | 7 Min. | 25 Min. |

Beispiel 3

30 kg Dipyridamol, 30 kg Fumarsäure, 29 kg Polyacrylsäure (Handelsname Carbopol 940), 1 kg Magnesiumstearat werden exakt nach Beispiel 2 in ein Granulat überführt.

Wirkstofffreigabe Dipyridamol:

| pH-Wert | 50% | >90% |
|---------|---------|---------|
| 1,2 | 12 Min. | 25 Min. |
| 4,0 | 15 Min. | 40 Min. |

Beispiel 4

2,5 kg Dipyridamol, 5,0 kg Fumarsäure, 1,3 kg hydriertes Rizinusöl (Handelsname Cutina HR), 0,1 kg pyrogene Kieselsäure (Handelsname Aerosil), werden exakt nach Beispiel 1 in ein Granulat überführt.

Wirkstofffreigabe Dipyridamol:

| pH-Wert | 50% | 75% | >90% |
|---------|---------|---------|---------|
| 1,2 | 28 Min. | 65 Min. | 105 Min. |
| 4,0 | 34 Min. | 72 Min. | 123 Min. |

Beispiel 5

Man mischt 8,5 kg Dipyridamol mit 9,0 kg Betainhydrochlorid und befeuchtet die Mischung mit 2,2 kg einer 10%igen isopropanolischen Polyvinylpyrrolidonlösung. Nach dem Trocknen bei 40°C und Sieben mischt man das Granulat mit 100 g Magnesiumstearat und 230 g pyrogener Kieselsäure (Aerosil). Die zu Tabletten verpresste Mischung bricht man zu einem Granulat; Verwendung findet die Fraktion 0,4-1,2 mm.

Wirkstofffreigabe Dipyridamol:

| pH-Wert | 50% | >90% |
|---|---|---|
| 1,2 | 5 Min. | 9 Min. |
| 4,0 | 7 Min. | 14 Min. |

Nahezu identische Werte erhält man bei Austausch von Betainhydrochlorid gegen Natriumhydrogensulfat.

Beispiel 6

200 kg Weinsäure in sphäroider Kristallform mit einer Teilchengrösse zwischen 0,5 und 0,8 mm werden in einem rotierenden Kessel mit einer 5%igen alkoholischen Hydroxypropylmethylcellulose-Lösung isoliert.

Jeweils nach Befeuchten mit einer 10%igen alkoholischen Polyvinylpyrrolidon-Lösung wird solange ein fein pulverisiertes Gemisch aus

Dipyridamol 8 Teile
Fumarsäure 2 Teile

eingestreut, bis die Pellets wieder frei laufen. Nach einer kurzen Trockenphase sprüht man erneut Kleberlösung und trägt danach weiteres Pulver ein. Insgesamt werden auf diese Weise 150 kg der Pulvermischung eingetragen, wozu ca. 75 kg Kleberlösung notwendig sind. Die Wirkstoffpellets sind zwischen 0,6 und 0,9 mm gross und enthalten 33% Wirkstoff und 64% organische Säure. Die Pellets werden nach der letzten Pulverauftragung gut getrocknet.

Wirkstofffreigabe Dipyridamol:

| pH-Wert | 50% | >90% |
|---|---|---|
| 1,2 | 2 Min. | 3 Min. |
| 4,0 | 2 Min. | 3 Min. |
| 6,0 | 2 Min. | 4 Min. |

Als Starterkerne wurden ausserdem Zuckerkügelchen (Nonpareille) oder folgende Säuren bzw. sauer reagierende Stoffe eingesetzt: Zitronensäure, Ascorbinsäure, Äpfelsäure, Bernsteinsäure, Natriumhydrogensulfat, Betainhydrochlorid, Mononatrium- oder Kaliumsalze der genannten mehrbasischen organischen Säuren.

Als saure Komponente der aufzutragenden Pulvermischung können neben Fumarsäure alle genannten sauer reagierenden Stoffe eingesetzt werden, bevorzugt sind jedoch Fumarsäure, Bernsteinsäure und Betainhydrochlorid. Es können auch Mischungen dieser sauren Stoffe verwendet werden.

Das Verhältnis des auf die Kerne aufzutragenden Gemisches aus Dipyridamol und sauer reagierender Komponente kann neben dem oben genannten von 8:2 auch folgende Werte aufweisen: 10:0; 9:1; 7:3; 6:4; 5:5; 4:6; 3:7; 2:8;1:9. Auch die Menge des auf die Starter aufzutragenden Pulvergemisches kann variiert werden. Sicherzustellen ist jedoch, dass bei den Pellets insgesamt auf 1 Mol Dipyridamol mindestens 5 Val saure Hilfsstoffe eingesetzt werden.

Beispiel 7

In einem Wirbelschichtgranulator werden 14 kg Dipyridamol, 0,5 kg pyrogene Kieselsäure (Handelsname Aerosil), 0,7 kg Maisstärke, 2,5 kg Polyäthylenglykolpulver 6000 und 25 kg Bernsteinsäure bei 70°C, 2 Stunden gewirbelt. Nach dem Abkühlen siebt man durch ein 1,0 mm Sieb, mischt 0,4% Magnesiumstearat zu und füllt in Hartgelatine-Steckkapseln ab.

Wirkstofffreigabe Dipyridamol aus Hartgelatine-Steckkapseln:

| pH-Wert | 50% | >90% |
|---|---|---|
| 1,2 | 9 Min. | 18 Min. |
| 4,0 | 12 Min. | 22 Min. |

Anstelle von Bernsteinsäure finden auch Verwendung Fumarsäure, Natriumhydrogensulfat und Natriumhydrogentartrat. Das Verhältnis Dipyridamol-Säure bzw. saures Salz kann durch Änderung der Zusammensetzung der Mischung innerhalb der genannten Grenze liegen.

Beispiel 8

1,9 kg Dipyridamol-Wirkstoffpellets nach Beispiel 6 werden in einem rasch umlaufenden Dragierkessel mit Schikanen mit einer 10%igen Lösung von Hydroxypropylmethylcellulosephthalat (Handelsname HP 55) in Aceton/Isopropanol 1:1 besprüht. Als Weichmacher wird Triacetin zugesetzt:

Die Wirkstofffreigabe bei 4 Gew.% Hülle ist folgendermassen:

| pH-Wert | 25% | 50% | 75% | 90% |
|---|---|---|---|---|
| 1,2 | 19 Min. | 40 Min. | 63 Min. | 87 Min. |
| 4,0 | 17 Min. | 35 Min. | 64 Min. | 90 Min. |
| 5,0 | 14 Min. | 30 Min. | 55 Min. | 85 Min. |

Beispiel 9

Es wird ein Granulat hergestellt aus 5 kg Dipyridamol, 2,5 kg Polyäthylenglykolpulver 6000, 3 kg Fumarsäure, 2 kg Weinsäure und 0,3 kg pyrogene Kieselsäure (Handelsname Aerosil). Nach dem Sieben mischt man 0,2 kg Magnesiumstearat zu und verpresst zu 10 mm bikonvexen Kernen mit 260 mg Gewicht = 100 mg Dipyridamol/Kern. Nach gründlichem Entstauben werden die Kerne in einem Drageekessel mit einer Diffusionsmembran versehen. Sie werden mit einer Lösung von
Methacrylsäure-Methacrylsäureester
Mischpolymerisat (Eudragit S®)
und
Hydroxypropylmethylcellulosephthalat (HP 55®)
im Verhältnis 2:8 besprüht (Aceton/Isopropanol 1:1 ). Bezogen auf die Lacktrockensubstanz wird 25% Polyäthylenglykol 6000 als Weichmacher und, falls gewünscht, Farblack und Talkum zugesetzt.

Bei 4 Gew.% Hüllenanteil erhält man folgende Freigabedaten:

| pH-Wert | 25% | 50% | 75% | 90% |
|---|---|---|---|---|
| 1,2 | 25 Min. | 44 Min. | 68 Min. | 95 Min. |
| 4,0 | 30 Min. | 48 Min. | 74 Min. | 105 Min. |
| 6,0 | 27 Min. | 39 Min. | 54 Min. | 77 Min. |

Beispiel 10

Dipyridamol-Filmtabletten mit 80 mg Weinsäure/Tablette
1 Tablette enthält:

| Dipyridamol | 75 mg oder |
| Dipyridamol-hydrochlorid | 80 mg |
| Weinsäure | 80 mg |
| Tablettenhilfsstoffe ad | 250 mg |

Herstellung:

Der Wirkstoff wird mit den Tablettenhilfsstoffen mit Ausnahme des Schmiermittels wässrig granuliert. Dem fertigen Granulat wird das Schmiermittel sowie die mit Polyvinylpyrrolidon und Talkum umhüllte Weinsäure zugemischt = pressfertige Mischung. Aus ihr werden runde, bikonvexe Presslinge von 8 mm Durchmesser hergestellt. Diese werden zur Geschmacksabdeckung mit einem Hydroxypropylmethylcellulose-Überzug versehen.

Beispiel 11

Dipyridamol-Filmtabletten mit 60 mg Fumarsäure/Tablette
1 Tablette enthält:

| Dipyridamol | 75 mg oder |
| Dipyridamol-hydrochlorid | 80 mg |
| Fumarsäure | 60 mg |
| Tablettenhilfsstoffe ad | 195 mg |

Herstellung:

Dipyridamol wird mit Fumarsäure vermischt und feucht granuliert. Dem fertigen Granulat werden die restlichen direkt tablettierbaren Hilfsstoffe zugemischt = pressfertige Mischung. Aus ihr werden runde, bikonvexe Kerne von 8 mm Durchmesser gepresst und diese zur Geschmacksabdeckung mit einem Hydroxypropylmethylcellulose-Überzug im Dragierkessel versehen.

Beispiel 12

Dipyridamol-Filmtabletten mit 120 mg Fumarsäure/Tablette
1 Tablette enthält:

| Dipyridamol | 75 mg |
| Fumarsäure | 120 mg |
| Tablettenhilfsstoffe ad | 225 mg |

Herstellung:

Analog Dipyridamol-Filmtabletten mit 60 mg Fumarsäure.

Beispiel 13

Dipyridamol-Filmtabletten mit 60 mg Fumarsäure/Tablette
1 Tablette enthält:

| Dipyridamol | 75,0 mg |
| Tablettenhilfsstoffe | 60,0 mg |
| Fumarsäure | 60,0 mg |

Herstellung:

Dipyridamol wird abweichend von Beispiel 11 mit üblichen Tablettenhilfsstoffen granuliert. Diesem Granulat wird die Fumarsäure und das Schmiermittel zugemischt = pressfertige Mischung. Die weitere Verarbeitung erfolgt analog Beispiel 11.

Beispiel 14

Dipyridamol-Filmtabletten mit 60 mg Fumarsäure/Tablette

10

1 Tablette enthält:

| | |
|---|---|
| Dipyridamol | 75,0 mg |
| Tablettenhilfsstoffe | 60,0 mg |
| Fumarsäure | 60,0 mg |

Nach Beispiel 2 wird aus 75 kg Dipyridamol, 60 kg Fumarsäure und 60 kg direkt tablettierbaren Hilfsstoffen (u.a. Polyvinylpyrrolidon, Magnesiumstearat) ein Granulat trocken hergestellt = pressfertige Mischung. Hieraus werden gemäss Beispiel 11 Kerne von 8,0 mm Durchmesser gepresst, die mit einem geschmacksabdeckenden Überzug versehen werden.

## Patentansprüche

### Anspruch für folgenden Vertragsstaaten : BE, CH, DE, FR, IT, LI, LU, NL, SE

1. Verwendung von Pellets und Granulaten, bestehend aus Dipyridamol oder dessen Säueaddiditonssalz und einem oral verträglichen sauren Hilfsstoff in inniger Vermischung und, gegebenenfalls, weiteren üblichen Zusatzstoffen, wobei auf 1 Mol Dipyridamol oder 1 Mol von dessen Säureadditionssalz 5 bis 30 Val eines oral verträglichen sauren Hilfsstoffes kommen, zur Herstellung von fertigen Arzneiformen mit rascher Wirkstoff-Freigabe, wobei diese Arzneiformen als Pellets oder Granulate in geschmacksabdeckenden Kapseln abgefüllt sind oder in Form von mit einem geschmacksabdeckenden Überzug versehenen Pellets, Granulate oder Tabletten vorliegen.

2. Orale Dipyridamolformen in Form von Granulaten oder Pellets mit einer relativen Bioverfügbarkeit von über 100 % bezogen auf Dipyridamollösungen und wesentlich verringerten inter- und intraindividuellen Blutspiegelschwankungen, dadurch gekennzeichnet, daß diese Formen auf 1 Mol Dipyridamol oder auf 1 Mol von dessen Säureadditionssalz 5 bis 30 Val eines oral verträglichen sauren Hilfsstoffes, gegebenenfalls neben üblichen Zusatzstoffen, enthalten, wobei Wirkstoff und saurer Hilfsstoff innig miteinander vermischt und die daraus gebildeten Granulate und Pellets in geschmacksabdeckenden Kapseln abgefüllt sind.

3. Pellets und Granulate zur Herstellung von fertigen Arzneiformen gemäß Anspruch 1 und 2, dadurch gekennzeichnet, daß diese Formen auf 1 Mol Dipyridamol oder auf 1 Mol von dessen Säureadditionssalz 10 bis 30 Val eines oral verträglichen sauren Hilfsstoffes enthalten.

4. Pellets und Granulate zur Herstellung von fertigen Arzneiformen gemäß Anspruch 1, 2 und 3, dadurch gekennzeichnet, daß als saure Hilfsstoffe Weinsäure, Zitronensäure, Fumarsäure, Bernsteinsäure, Äpfelsäure, Ascorbinsäure, Adipinsäure, die sauren Natrium- und Kaliumsalze dieser Säuren, Natrium- oder Kaliumhydrogensulfat, Betainhydrochlorid, die in Wasser zu Säuren hydrolysierenden Anhydride der Bernsteinsäure, Glutarsäure oder das D-Glucuronsäure-$\gamma$-lacton oder Gemische dieser sauren Hilfsstoffe eingesetzt werden.

5. Pellets und Granulate zur Herstellung von fertigen Arzneiformen gemäß den Ansprüchen 1 bis 4, dadurch gekennzeichnet, daß Fumarsäure als saurer Hilfsstoff eingesetzt wird.

6. Pellets und Granulate zur Herstellung von fertigen Arzneiformen gemäß den Ansprüchen 1 bis 5, dadurch gekennzeichnet, daß in diesen Formen das Dipyridamol mit den sauren Hilfsstoffen innig vermischt vorliegt, die Granulate einen partikeldurchmesser von 0,1 bis 2,0 mm, vorzugsweise 0,25 bis 1,25 mm, die Pellets einen Partikeldurchmesser von 0,1 bis 2,0 mm, vorzugsweise 0,5 bis 1,5 mm, aufweisen und die Pellets oder Granulate in Hartgelatinekapseln abgefüllt sind.

7. Pellets und Granulate zur Herstellung von fertigen Arzneiformen gemäß den Ansprüchen 1 bis 6, dadurch gekennzeichnet, daß dem Dipyridamol oder dessen Säureadditionssalz und den sauren Hilfsstoffen noch wasserlösliche Bindemittel und/oder schleimbildende Hilfsstoffe und/oder wasserabstoßende Hilfsstoffe zugemischt sind.

8. Granulate zur Herstellung von fertigen Arzneiformen gemäß Anspruch 7, dadurch gekennzeichnet, daß die Mischungen, bestehend aus Wirkstoff, sauren Hilfsstoffen und anderen üblichen Hilfsstoffen, in Gegenwart von Methyl-, Ethyl-, Hydroxyethyl- oder Hydroxypropylmethylcellulose oder Polyacrylsäuren oder von Fetten verpreßt und granuliert sind.

9. Verwendung von Pellets und Granulaten zur Herstellung von fertigen Arzneiformen gemäß den Ansprüchen 1 und 3 bis 8, dadurch gekennzeichnet, daß der Wirkstoff mit einem oder mehreren sauren Hilfsstoffen sowie weiteren direkttablettierbaren Hilfsstoffen sowie dem Schmiermittel zur einer direkttablettierbaren Mischung verarbeitet und diese nach Verpressung zu Tablettenkernen mit einem geschmacksabdeckenden Überzug versehen ist.

10. Verwendung von Granulaten zur Herstellung von fertigen Arzneiformen gemäß den Ansprüchen 1 und 3 bis 8, dadurch gekennzeichnet, daß der Wirkstoff mit einem bzw. mehreren sauren Hilfsstoffen durch Feucht- bzw. Trockengranulation in ein Granulat überführt wurde und dieses nach Zumischung weiterer Hilfsstoffe zu Kernen verarbeitet ist und diese mit einem geschmacksabdeckenden Überzug versehen sind.

11. Verwendung von Granulaten zur Herstellung von fertigen Arzneiformen gemäß den Ansprüchen 1 und 3 bis 8, dadurch gekennzeichnet, daß der Wirkstoff mit üblichen Hilfsstoffen durch Feucht- oder Trockengranulation in ein Granulat überführt wurde und dieses nach Zumischung von einem oder mehreren sauren Hilfsstoffen sowie dem Schmiermittel zu Kernen verpreßt ist und diese mit einem geschmacksabdeckenden Überzug versehen sind.

## Ansprüche für folgenden Vertragsstaat: AT

1. Verfahren zur Herstellung von neuen oralen Dipyridamolformen in Form von Granulaten Pellets oder Tabletten mit einer hohen Bioverfügbarkeit, dadurch gekennzeichnet, daß 1 Mol Dipyridamol oder dessen Säureadditionssalz mit 5 bis 30 Val eines oral verträglichen sauren Hilfsstoffes innig vermischt und gegebenenfalls zusätzlich mit üblichen Zusatzstoffen zu Pellets oder zu Granulaten verarbeitet werden und die so erhaltenen Granulate bzw. Pellets abgefüllt oder zu Tabletten verpreßt werden.

2. Verfahren gemäß Anspruch 1, dadurch gekennzeichnet, daß auf 1 Mol Dipyridamol oder auf 1 Mol von dessen Säureadditionssalz 10 bis 30 Val eines oral verträglichen sauren Hilfsstoffes verwendet werden.

3. Verfahren gemäß Anspruch 1 und 2, dadurch gekennzeichnet, daß als saure Hilfsstoffe Weinsäure, Zitronensäure, Fumarsäure, Bernsteinsäure, Äpfelsäure, Ascorbinsäure, Adipinsäure, die sauren Natrium- und Kaliumsalze dieser Säuren, Natrium- oder Kaliumhydrogensulfat, Betainhydrochlorid, die in Wasser zu Säuren hydrolysierenden Anhydride der Bernsteinsäure, Glutarsäure oder das D-Glucuronsäure-γ-lacton oder Gemische dieser sauren Hilfsstoffe, vorzugsweise jedoch Fumarsäure, verwendet werden.

4. Verfahren gemäß den Ansprüchen 1 bis 3, dadurch gekennzeichnet, daß Granulate mit einer Partikelgröße zwischen 0,1 und 2,0 mm, vorzugsweise 0,25 und 1,25 mm, Durchmesser hergestellt und gegebenenfalls in Hartgelatinekapseln abgefüllt werden.

5. Verfahren gemäß den Ansprüchen 1 bis 3, dadurch gekennzeichnet, daß Pellets mit einer Partikelgröße zwischen 0,1 und 2,0 mm, vorzugsweise 0,5 bis 1,5 mm, Durchmesser hergestellt und gegebenenfalls in Hartgelatinekapseln abgefüllt werden.

6. Verfahren gemäß den Ansprüchen 1 bis 5, dadurch gekennzeichnet, daß als weitere Zusatzstoffe noch wasserlösliche Bindemittel und/oder schleimbildende Hilfsstoffe und/oder wasserabstoßende Hilfsstoffe zugemischt werden.

7. Verfahren gemäß Anspruch 6, dadurch gekennzeichnet, daß die Mischungen in Gegenwart von Methyl-, Äthyl-, Hydroxyäthyl- oder Hydroxypropylmethylcellulose oder Polyacrylsäuren oder von Fetten verpreßt und granuliert werden.

8. Verfahren gemäß den Ansprüchen 1 bis 7, dadurch gekennzeichnet, daß Dipyridamol oder dessen Säureadditionssalz mit einem oder mehreren sauren Hilfsstoffen sowie weiteren direkttablettierbaren Hilfsstoffen sowie dem Schmiermittel zu einer direkttablettierbaren Mischung verarbeitet wird, die anschließend zu Tablettenkernen verpreßt wird.

9. Verfahren gemäß den Ansprüchen 1 bis 7, dadurch gekennzeichnet, daß Dipyridamol oder dessen Säureadditionssalz mit einem bzw. mehreren sauren Hilfsstoffen durch Feucht- bzw. Trockengranulation in ein Granulat überführt wird und dieses nach Zumischung weiterer Hilfsstoffe zu Kernen verpreßt wird.

10. Verfahren gemäß den Ansprüchen 1 bis 7, dadurch gekennzeichnet, daß Dipyridamol oder dessen Säureadditionssalz mit üblichen Hilfsstoffen durch Feucht- oder Trockengranulation in ein Granulat überführt und dieses nach Zumischung von einem oder mehreren sauren Hilfsstoffen sowie dem Schmiermittel zur Kernen verpreßt wird.

## Revendications

### Revendications pour les Etats contractants suivants: BE, CH, DE, FR, IT, LI, LU, NL, SE

1. Utilisation de pastilles et de granulés constitués de dipyridamole ou de son sel d'addition d'acide et d'un adjuvant acide compatible par voie orale en mélange intime ainsi qu'éventuellement d'autres adjuvants usuels, 5 à 30 val d'un adjuvant acide compatible par voie orale étant mis en oeuvre pour 1 mole de dipyridamole ou pour 1 mole de son sel d'addition d'acide, pour la préparation de formes médicamenteuses finies avec libération rapide de la substance active, ces formes médicamenteuses sous forme de pastilles ou de granulés étant chargées dans des capsules masquant le goût ou se présentant sous forme de pastilles, de granulés ou de comprimés munis d'un enrobage masquant le goût.

2. Formes orales de dipyridamole sous forme de granulés ou de pastilles présentant une biodisponibilité

EP 0 068 191 B2

relative supérieure à 100% rapportés aux solutions de dipyridamole ainsi que des variations de taux sanguin inter- et intra-individuelles essentiellement réduites, caractérisées en ce que ces formes contiennent pour 1 mole de dipyridamole ou pour 1 mole de son sel d'addition d'acide 5 à 30 val d'un adjuvant acide compatible par voie orale, éventuellement en présence d'additifs usuels, la substance active et l'adjuvant acide étant mélangés intimement l'un avec l'autre et les granulés et pastilles en résultant étant chargés dans des capsules masquant le goût.

3. Pastilles et granulés pour la préparation de formes médicamenteuses finies selon les revendications 1 et 2, caractérisés en ce que ces formes contiennent pour 1 mole de dipyridamole ou pour 1 mole de son sel d'addition d'acide 10 à 30 val d'un adjuvant acide compatible par voie orale.

4. Pastilles et granulés pour la préparation de formes médicamenteuses finies selon les revendications 1, 2 et 3, caractérisés en ce que l'on utilise en tant qu'adjuvants acides, l'acide tartrique, l'acide citrique, l'acide fumarique, l'acide succinique, l'acide malique, l'acide ascorbique, l'acide adipique, les sels de sodium et de potassium acides de ces acides, l'hydrogénosulfate de sodium ou de potassium, le chlorhydrate de bétaïne, les anhydrides de l'acide succinique, de l'acide glutarique hydrolysables dans l'eau en acides ou la γ-lactone de l'acide D-glucuronique ou des mélanges de ces adjuvants acides.

5. Pastilles et granulés pour la préparation de formes médicamenteuses finies selon les revendications 1 à 4, caractérisés en ce que l'on utilise l'acide fumarique en tant qu'adjuvant acide.

6. Pastilles et granulés pour la préparation de formes médicamenteuses finies selon les revendications 1 à 5, caractérisés en ce que, dans ces formes, le dipyridamole est présent en mélange intime avec les adjuvants acides, que les granulés ont un diamètre de particule de 0,1 à 2,0 mm, de préférence de 0,25 à 1,25 mm, que les pastilles ont un diamètre de particule de 0,1 à 2,0 mm, de préférence de 0,5 à 1,5 mm et que les pastilles ou les granulés sont chargés dans des capsules en gélatine dure.

7. Pastilles et granulés pour la préparation de formes médicamenteuses finies selon les revendications 1 à 6, caractérisés en ce que des liants hydrosolubles et/ou des adjuvants formant du mucilage et/ou des adjuvants hydrophobes sont en outre ajoutés par mélange au dipyridamole ou à son sel d'addition d'acide et aux adjuvants acides.

8. Granulés pour la préparation de formes médicamenteuses finies selon la revendication 7, caractérisés en ce que les mélanges constitués de substance active, d'adjuvants acides et d'autres adjuvants usuels sont pressés et granulés en présence de méthyl-, éthyl-, hydroxyéthyl- ou hydroxypropylméthylcellulose ou d'acides polyacryliques ou de graisses.

9. Utilisation de pastilles et de granulés pour la préparation de formes médicamenteuses finies selon les revendications 1 et 3 à 8, caractérisée en ce que la substance active est transformée avec un ou plusieurs adjuvants acides ainsi qu'avec d'autres adjuvants formulables directement sous forme de comprimés ainsi qu'avec du lubrifiant en un mélange transformable directement en comprimés et que celui-ci est muni, après pressage en des noyaux de comprimés, d'un enrobage masquant le goût.

10. Utilisation de granulés pour la préparation de formes médicamenteuses finies selon les revendications 1 et 3 à 8, caractérisée en ce que la substance active a été transformée en granulés, avec un ou plusieurs adjuvants acides, par granulation par voie humide ou sèche, que ces granulés sont transformés en noyaux après mélange avec d'autres adjuvants et que ces noyaux sont munis d'un enrobage masquant le goût.

11. Utilisation de granulés pour la préparation de formes médicamenteuses finies selon les revendications 1 et 3 à 8, caractérisée en ce que la substance active a été transformée en granulés, avec des adjuvants usuels, par granulation par voie humide ou sèche et que ces granulés sont pressés, après incorporation d'un ou de plusieurs adjuvants acide ainsi que du lubrifiant, en des noyaux et que ceux-ci sont munis d'un enrobage masquant le goût.

**Revendications pour l'Etat contractant suivant: AT**

1. Procédé pour la préparation de nouvelles formes orales de dipyridamole sous forme de granulés, pastilles ou comprimés présentant une biodisponibilité élevée, caractérisé en ce que 1 mole de dipyridamole ou de son sel d'addition d'acide est intimement mélangée avec 5 à 30 val d'un adjuvant acide compatible par voie orale et est en outre transformée le cas échéant, en pastilles ou en granulés avec des adjuvants usuels et que les granulés ou pastilles ainsi obtenus sont chargés ou pressés en comprimés.

2. Procédé selon la revendication 1, caractérisé en ce que l'on utilise pour 1 mole de dipyridamole ou pour 1 mole de son sel d'addition d'acide 10 à 30 val d'un adjuvant acide compatible par voie orale.

3. Procédé selon les revendications 1 et 2, caractérisé en ce que l'on utilise en tant qu'adjuvants acides, l'acide tartrique, l'acide citrique, l'acide fumarique, l'acide succinique, l'acide malique, l'acide ascorbique, l'acide adipique, les sels de sodium et de potassium acides de ces acides, l'hydrogénosulfate de sodium ou de potassium, le chlorhydrate de bétaïne, les anhydrides de l'acide succinique, de l'acide glutarique hydroly-

13

sables dans l'eau en acides ou la γ -lactone de l'acide D-glucuronique ou des mélanges de ces adjuvants acides, avec toutefois une préférence pour l'acide fumarique.

4. Procédé selon les revendications 1 à 3, caractérisé en ce que l'on prépare des granulés avec une taille de particule entre 0,1 et 2,0 mm, de préférence entre environ 0,25 et 1,25 mm de diamètre et les introduit éventuellement dans des capsules en gélatine dure.

5. Procédé selon les revendications 1 à 3, caractérisé en ce que l'on prépare des pastilles avec une taille de particule entre 0,1 et 2,0 mm, de préférence entre 0,5 et 1,5 mm de diamètre et les introduit éventuellement dans des capsules en gélatine dure.

6. Procédé selon les revendications 1 à 5, caractérisé en ce qu'on ajoute en outre par mélange, en tant qu'adjuvants additionnels, des liants hydrosolubles et/ou des adjuvants formant du mucilage et/ou des adjuvants hydrophobes.

7. Procédé selon la revendication 6, caractérisé en ce que les mélanges sont pressés et granulés en présence de méthyl-, éthyl-, hydroxyéthyl- ou hydroxypropylméthylcellulose ou d'acides polyacryliques ou de graisses.

8. Procédé selon les revendications 1 à 7, caractérisé en ce que du dipyridamole ou son sel d'addition d'acide est transformé avec un ou plusieurs adjuvants acides ainsi qu'avec d'autres adjuvants formulables directement sous forme de comprimés ainsi qu'avec le lubrifiant en un mélange transformable directement en comprimés, lequel est ensuite pressé en des noyaux de comprimés.

9. Procédé selon les revendications 1 à 7, caractérisé en ce que du dipyridamole ou son sel d'addition d'acide est transformé en granulés, avec un ou plusieurs adjuvants acides, par granulation par voie humide ou sèche et que ces granulés sont pressés en noyaux après mélange avec d'autres adjuvants.

10. Procédé selon les revendications 1 à 7, caractérisé en ce que du dipyridamole ou son sel d'addition d'acide est transformé en granulés, avec des adjuvants usuels, par granulation par voie humide ou sèche et que ces granulés sont pressés en noyaux après incorporation d'un ou de plusieurs adjuvants acides ainsi que du lubrifiant.


## Claims

**Claims for the following Contracting States: BE, CH, DE, FR, IT, LI, LU, NL, SE**

1. Use of pellets and granulates consisting of dipyridamole or an acid addition salt thereof and an orally acceptable acidic excipient intimately mixed together and, optionally, other conventional additives, there being 5 to 30 equivalents of an orally acceptable acidic excipient to 1 mol of dipyridamole or 1 mol of an acid addition salt thereof, for the production of complete pharmaceutical preparations with rapid release of the active substance, these pharmaceutical preparations being packed in the form of pellets or granulates in flavour-masking capsules or being present in the form of pellets, granules or tablets provided with a flavour-masking coating.

2. Oral dipyridamole preparations in the form of granulates or pellets having a relative bioavailability of more than 100%, based on dipyridamole solutions, and substantially reduced inter- and intra-individual blood level fluctuations, characterised in that these preparations contain to 1 mol of dipyridamole or to 1 mol of an acid addition salt thereof 5 to 30 equivalents of an orally acceptable acidic excipient, optionally together with conventional additives, the active substance and acidic excipient being intimately mixed together and the granulates and pellets formed therefrom being packed in flavour-masking capsules.

3. Pellets and granulates for the production of complete pharmaceutical preparations according to claims 1 and 2, characterised in that these preparations contain to 1 mol of dipyridamole or to 1 mol of an acid addition salt thereof 10 to 30 equivalents of an orally acceptable acidic excipient.

4. Pellets and granulates for the production of complete pharmaceutical preparations according to claims 1, 2 and 3, characterised in that the acidic excipients used are tartaric, citric, fumaric, succinic, malic, ascorbic or adipic acid, the acid sodium and potassium salts of these acids, sodium or potassium hydrogen sulphate, betaine hydrochloride, the anhydrides of succinic or glutaric acid which hydrolyse in water to form acids, or D-glucuronic-γ-lactone or mixtures of these acidic excipients.

5. Pellets and granulates for the production of complete pharmaceutical preparations according to claims 1 to 4, characterised in that fumaric acid is used as the acidic excipient.

6. Pellets and granulates for the production of complete pharmaceutical preparations according to claims 1 to 5, characterised in that, in these preparations, the dipyridamole is intimately mixed with the acidic excipients, the granulates have a particle diameter of 0.1 to 2.0 mm, preferably 0.25 to 1.25 mm, the pellets have a particle diameter of 0.1 to 2.0 mm, preferably 0.5 to 1.5 mm, and the pellets or granulates are packed into hard gelatine capsules.

7. Pellets and granulates for the production of complete pharmaceutical preparations according to claims 1 to 6, characterised in that water-soluble binders and/or mucilaginous excipients and/or water-repellent excipients are added to the dipyridamole or the acid addition salt thereof and the acidic excipients.

8. Granulates for the production of complete pharmaceutical preparations according to claim 7, characterised in that the mixtures consisting of active substance, acidic excipients and other conventional excipients are compressed and granulated in the presence of methyl-, ethyl-, hydroxyethyl- or hydroxypropylmethyl cellulose or polyacrylic acids or fats.

9. Use of pellets and granulates for the production of complete pharmaceutical preparations according to claims 1 and 3 to 8, characterised in that the active substance is combined with one or more acidic excipients and other excipients which can be incorporated directly in tablets and with the lubricant to form a mixture which can be made directly into tablets and this mixture is then compressed to form tablet cores which are then coated with a flavour-masking coating.

10. Use of granulates for the production of complete pharmaceutical preparations according to claims 1 and 3 to 8, characterised in that the active substance is converted into a granulate by moist or dry granulation with one or more acidic excipients and, after the addition of further excipients, this granulate is made into cores and these are coated with a flavour-masking coating.

11. Use of granulates for the production of complete pharmaceutical preparations according to claims 1 and 3 to 8, characterised in that the active substance is converted into a granulate by moist or dry granulation with conventional excipients and, after the addition of one or more acidic excipients and the lubricant, this granulate is compressed to form cores and these are then provided with a flavour-masking coating.

## Claims for the following Contracting State: AT

1. Process for producing new oral dipyridamole preparations in the form of granulates, pellets or tablets with a high bioavailability, characterised in that 1 mol of dipyridamole or an acid addition salt thereof is intimately mixed with 5 to 30 equivalents of an orally acceptable acidic excipient and optionally also processed with conventional additives to form pellets or granulates and the resulting granulates or pellets are packed into containers or compressed to form tablets.

2. Process according to claim 1, characterised in that to 1 mol of dipyridamole or to 1 mol of the acid addition salt thereof 10 to 30 equivalents of an orally acceptable acidic excipient are used.

3. Process according to claims 1 and 2, characterised in that the acidic excipients used are tartaric, citric, fumaric, succinic, malic, ascorbic or adipic acid, the acid sodium and potassium salts of these acids, sodium or potassium hydrogen sulphate, betaine hydrochloride, the anhydrides of succinic or glutaric acid which hydrolyse in water to form acids, or D-glucuronic acid-γ-lactone or mixtures of these acidic excipients, but preferably fumaric acid.

4. Process according to claims 1 to 3, characterised in that granulates having a particle size of between 0.1 and 2.0 mm, preferably 0.25 and 1.25 mm in diameter are produced and optionally packed into hard gelatine capsules.

5. Process according to claims 1 to 3, characterised in that pellets with a particle size of between 0.1 and 2.0 mm, preferably 0.5 to 1.5 mm diameter are produced and are optionally packed into hard gelatine capsules.

6. Process according to claims 1 to 5, characterised in that water-soluble binders and/or mucilaginous excipients and/or water-repellent excipients are also mixed in as further additives.

7. Process according to claim 6, characterised in that the mixtures are compressed and granulated in the presence of methyl-, ethyl-, hydroxyethyl- or hydroxy-propylmethylcellulose or polyacrylic acids or fats.

8. Process according to claims 1 to 7, characterised in that dipyridamole or an acid addition salt thereof is processed with one or more acidic excipients and other excipients which can be incorporated directly into tablets and with the lubricant to form a mixture which can be made directly into tablets and this mixture is then compressed to form tablet cores.

9. Process according to claims 1 to 7, characterised in that dipyridamole or an acid addition salt thereof is made into a granulate with one or more acidic excipients by moist or dry granulation and, after the addition of further excipients, this granulate is compressed to form cores.

10. Process according to claims 1 to 7, characterised in that dipyridamole or an acid addition salt thereof is converted into a granulate by moist or dry granulation with conventional excipients and, after the addition of one or more acidic excipients and the lubricant, this granulate is compressed to form cores.

ABB. 1

ABB. 2

PLASMASPIEGEL DIPYRIDAMOL

MCG/ML

● = NEUE FORM (BEISP.6)

■ = NEUE FORM (BEISP.8)

✗ = HANDELSFORM

ZEIT (STD.)

EP 0 068 191 B2

PLASMASPIEGEL DIPYRIDAMOL     ABB. 3

FORMENVERGLEICH BEI EINZELPROBANDEN

18

ABB. 4

DIPYRIDAMOL PLASMASPIEGEL
VERSCHIEDENE DOSIERUNGEN

● = Neue Form 75 mg, Beispiel 6

■ = Neue Form 50 mg, Beispiel 6

X = Handelsform Dragee, 75 mg

MCG/ML

ZEIT (STD.)

EP 0 068 191 B2